(19) Europäisches Patentamt
European Patent Office
Office européen des brevets

(11) **EP 3 494 390 B1**

(12) **EUROPÄISCHE PATENTSCHRIFT**

(45) Veröffentlichungstag und Bekanntmachung des Hinweises auf die Patenterteilung:
**27.05.2020 Patentblatt 2020/22**

(21) Anmeldenummer: **17755075.3**

(22) Anmeldetag: **01.08.2017**

(51) Int Cl.:
***G01N 22/04*** *(2006.01)* ***G01N 33/36*** *(2006.01)*
***A61F 13/15*** *(2006.01)*

(86) Internationale Anmeldenummer:
**PCT/EP2017/069460**

(87) Internationale Veröffentlichungsnummer:
**WO 2018/024736 (08.02.2018 Gazette 2018/06)**

(54) **VERFAHREN ZUR MESSUNG VON ABSORBIERENDEN HYGIENEARTIKELN**

METHOD FOR MEASURING ABSORBENT HYGIENE ARTICLES

PROCÉDÉ DE MESURE D'ARTICLES D'HYGIÈNE ABSORBANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.08.2016 DE 102016114286**

(43) Veröffentlichungstag der Anmeldung:
**12.06.2019 Patentblatt 2019/24**

(73) Patentinhaber: **TEWS Elektronik GmbH & Co. KG 22459 Hamburg (DE)**

(72) Erfinder: **SCHLEMM, Udo 22607 Hamburg (DE)**

(74) Vertreter: **Hauck Patentanwaltspartnerschaft mbB Postfach 11 31 53 20431 Hamburg (DE)**

(56) Entgegenhaltungen:
**WO-A1-2014/170796 DE-A1-102010 026 178**

**Beschreibung**

Die vorliegende Erfindung betrifft ein Verfahren zur Messung von absorbierenden Hygieneartikeln, insbesondere zur Messung bei absorbierenden Hygieneartikeln während ihrer Herstellung, bei der auf eine fortlaufende Bahn voneinander beabstandete Saugkörper aufgebracht werden.

Aus EP 1 327 876 B1 ist ein Verfahren zum Erkennen von Fremdkörpern in kontinuierlichen Masseströmen aus faserförmigem, strangförmigem oder schüttgutartigem Material mit Hilfe eines Mikrowellenresonators bekannt geworden. Der Massestrom wird durch ein Feld des Mikrowellenresonators geführt, bei dem die durch das Material bewirkte Änderung (A) der Resonanzfrequenz und die Änderung (B) der Breite der Resonanzkurve des Mikrowellenresonators bestimmt wird. Das Verhältnis der Änderungen wird mit entsprechenden Mittelwerten verglichen und die Anwesenheit eines Fremdkörpers wird gemeldet, wenn das Verhältnis der Änderungen von dem Mittelwert um mehr als einen vorgegebenen Wert abweicht.

Aus WO 2014/170796 A1 ist ein Verfahren zur Herstellung von absorbierenden Hygieneartikeln bekannt. Der absorbierende Hygieneartikel, beispielsweise in Form einer Windel, weist ein absorbierendes Pad als Saugkörper auf, das wiederum eine oder mehrere absorbierende Materialen besitzt. Die absorbierenden Pads werden entlang einer Bahn eines durchlässigen Materials aufgebracht, wobei ein Mikrowellenresonator vorgesehen ist, der ein Gewichtsprofil oder ein Dichteprofil des mindestens einen absorbierenden Materials erstellt. Für die Messposition des Mikrowellenresonators werden unterschiedliche Positionen an der Windelmaschine vorgeschlagen. Besondere Probleme bei der Auswertung der Daten ergeben sich aus dem Umstand, dass bei der Herstellung von Windeln die Windelkerne mit geringem Abstand auf die endlose Bahn von PE-Außenhülle und permeabler Deckschicht aufgebracht werden. In diesem Sinne gibt es keinen Leerbetrieb des Mikrowellenresonators, der für einen Abgleich von Leerwerten genutzt werden könnte.

Der Erfindung liegt die Aufgabe zugrunde, ein Verfahren zur Messung von absorbierenden Hygieneartikeln mit Hilfe eines Mikrowellenresonators bereitzustellen.

Erfindungsgemäß wird die Aufgabe durch ein Verfahren mit den Merkmalen nach Anspruch 1 gelöst. Vorteilhafte Ausgestaltungen bilden den Gegenstand der Unteransprüche.

Das erfindungsgemäße Verfahren dient zur Messung von absorbierenden Hygieneartikeln, wobei bei dem Verfahren auf eine fortlaufende Bahn voneinander beabstandete Saugkörper aufgebracht werden. Feuchte und/oder Dichte der Saugkörper werden mit mindestens einem Mikrowellenresonator mit Hilfe einer Resonanzfrequenzverschiebung (A) und einer Resonanzfrequenzverbreiterung (B) erfasst. Bei dem erfindungsgemäßen Verfahren wird die Bahn durch den mindestens einen Mikrowellenresonator transportiert und fortlaufend werden zwei Mikrowellenwerte bestimmt, zu deren Auswertung jeweils ein Leerwert ($A_0$, $B_0$) von dem aktuellen Messwert subtrahiert wird. Das erfindungsgemäße Verfahren ist dadurch gekennzeichnet, dass zur Bestimmung der Leerwerte bei mindestens einem der Messwerte fortlaufend der Wert in einem lokalen Minimum bestimmt und mit einer Anzahl von zurückliegenden Werten für lokale Minima gemittelt wird, um als Leerwert von dem aktuellen Messwert subtrahiert zu werden. Bei dem erfindungsgemäßen Verfahren werden die Mikrowellenmesswerte fortlaufend bestimmt. Zur Auswertung werden die lokalen Minima ausgewertet, in denen auch die fortlaufende Bahn von dem Mikrowellenresonator erfasst wird. Da diese gemessenen Minima-Werte gewissen statistischen und auch systematischen Schwankungen unterliegen können, wird erfindungsgemäß auf einen gleitenden Mittelwert der lokalen Minima abgestellt. Mit dem gleitenden Mittelwert können beispielsweise eine vorbestimmte Anzahl der letzten Minima-Werte direkt oder gewichtet gemittelt werden.

In einer bevorzugten Weiterentwicklung des erfindungsgemäßen Verfahrens wird für einen der Messwerte die Position des lokalen Minimums bestimmt und der andere Messwert für die bestimmte Position ausgewertet und bei der Mittelwertbildung berücksichtigt. Alternativ dazu ist es möglich, für jeden der beiden Messwerte fortlaufend den Wert in einem lokalen Minimum zu ermitteln. Beide Ansätze haben Vor- und Nachteile. Handelt es sich bei dem zu untersuchenden Saugkörper auf der Bahn um ein Material, auf das nur einer der Mikrowellenmesswerte gut anspricht, so kann es von Vorteil sein, dass Minimum in dem anderen Signalwert nicht aus den zugehörigen Messwerten zu bestimmen, sondern auf die Position des lokalen Minimums für den empfindlicheren Messwert zurückzugreifen. Sind dagegen beide Mikrowellenmessgrößen annähernd gleich sensitiv für das zu detektierende Material, können beide lokalen Minima unabhängig voneinander gemessen und ausgewertet werden.

Für die Ermittlung des Leerwertes und der gemittelten Minima kann eine vorbestimmte Anzahl von zurückliegenden lokalen Minima geometrisch oder arithmetisch gemittelt werden. Das Ergebnis dieser Mittelung beschreibt einen mittleren Minimumswert, der von statistischen Schwankungen der Werte im Minimum weitgehend befreit ist.

Gerade bei der Herstellung von Windeln als absorbierenden Hygieneartikeln, aber auch bei anderen absorbierenden Hygieneartikeln kann das Problem auftreten, dass die fortlaufend zu vermessende Bahn für einen Mikrowellenresonator zu breit, bezogen auf die Transportrichtung, ausgefallen ist. Dieses Problem wird durch die Verwendung von mindestens zwei Mikrowellenresonatoren gelöst, die bezogen auf eine Transportrichtung der Bahn zueinander in Quer- und in Längsrichtung versetzt angeordnet sind, um die Bahn in ihrer gesamten Breite zu vermessen. Bezogen auf die Transportrichtung sind die zwei oder mehr Mikrowellenresonatoren in Querrichtung zueinander versetzt angeordnet, um die Bahn in ihrer gesamten Breite vermessen zu können. Hier ist es erforderlich, die quer zueinander versetzten Mikrowellenresonatoren auch in Längsrichtung zueinander versetzt anzuordnen, da diese in der Regel in einem zentralen Bereich eine homogene Feldverteilung besitzen. Durch den Versatz der Mikrowellenresonatoren in Längsrichtung können diese so überlappend zueinander angeordnet werden, dass die Bahn in ihrer gesamten Breite mit einer homogenen Feldverteilung vermessen wird.

In einer bevorzugten Weiterentwicklung des erfindungsgemäßen Messverfahrens bildet jeder Mikrowellenresonator einen Mittelwert seiner Messwerte in Querrichtung, bezogen auf die Transportrichtung der Bahn. Die gemittelten Messwerte der mehreren Mikrowellenresonatoren werden um einen Versatz der Mikrowellenresonatoren in Transportrichtung korrigiert, um einen Gesamtmittelwert für die gesamte Breite der Bahn zu bestimmen. Bei der Korrektur um den Versatz der Mikrowellenresonatoren wird berücksichtigt, dass aufgrund des räumlichen Versatzes der Mikrowellenresonatoren zueinander und der Transportgeschwindigkeit der Bahn die gemittelten Messwerte mit einem zeitlichen Versatz zueinander addiert werden müssen, um einen Mittelwert für die gesamte Breite der Bahn zu erhalten.

In einer bevorzugten Ausgestaltung besitzt jeder der Mikrowellenresonatoren einen Messbereich mit einem homogenen Feldverlauf, wobei die mehreren Mikrowellenresonatoren derart quer zur Transportrichtung positioniert sind, dass das gesamte Band von Messbereichen mit homogenem Feldverlauf überdeckt wird. Ein homogener Feldverlauf hilft, zuverlässige und genaue Messergebnisse zu erzielen. Durch den homogenen Feldverlauf ist es auch möglich, die aufgenommenen Messwerte in Querrichtung der Bahn zu mitteln. Mehrere Mikrowellenresonatoren werden derart hintereinander angeordnet, dass das gesamte Band insgesamt mit einem Messbereich mit homogenem Feldverlauf überdeckt ist.

In einer bevorzugten Ausgestaltung des erfindungsgemäßen Verfahrens wird ebenfalls die Masse eine Saugkörpers zwischen zwei lokalen Minima über ein Aufsummieren der Messwerte bestimmt. Hierbei wird bevorzugt jeder einzelne Messwert aufsummiert und in Relation mit der zu bestimmenden Gesamtmasse gesetzt.

Das erfindungsgemäße Verfahren wird nachfolgend an einem Ausführungsbeispiel näher erläutert. Es zeigen:

Fig. 1    den grundsätzlichen Aufbau einer Windelmaschine sowie mögliche Positionen für einen Mikrowellenresonator,

Fig. 2    Signale eines Windelstrangs, bei dem der Abstand der Windelkerne kleiner als ein Sensordurchmesser ist,

Fig. 3    Signale eines Windelstrangs, bei dem der Abstand der Windelkerne größer als der Windeldurchmesser ist,

Fig. 4    eine schematische Ansicht des Feldverlaufs in einem Gabelresonator in Längsrichtung und

Fig. 5    eine Anordnung von zwei Gabelsensoren bei einem bahnförmigen Messgut.

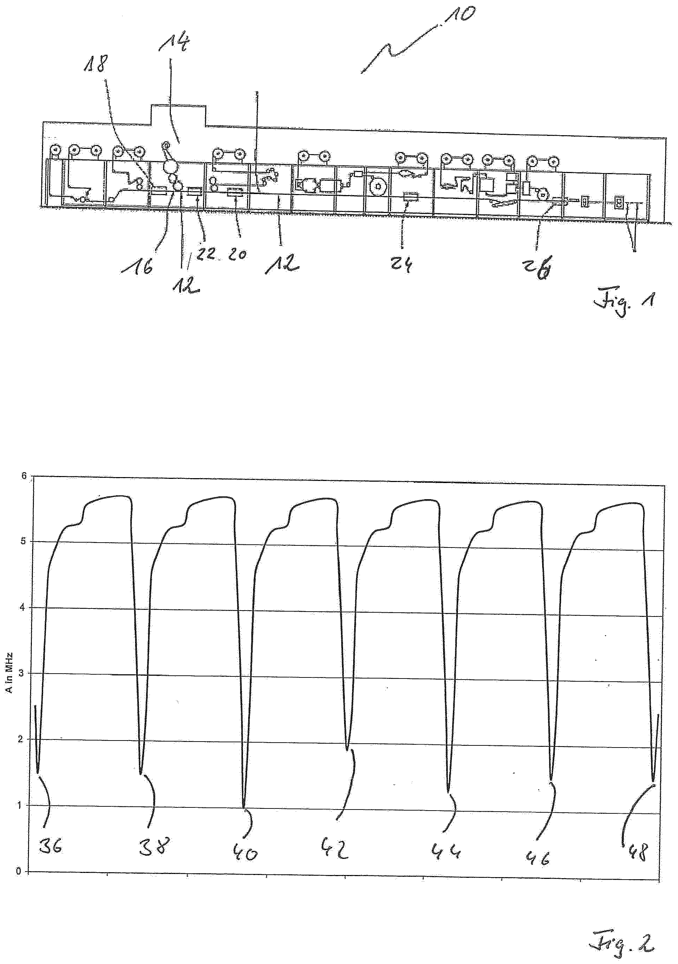

Fig. 1 zeigt in einer schematischen Ansicht den Gesamtaufbau einer Windelmaschine 10. Die Maschine 10 besitzt eine fortlaufende Bahn 12 aus einer PE-Außenhülle, die kontinuierlich mit großer Geschwindigkeit durch die Maschine läuft, um eine Windel herzustellen. In periodischen Abständen werden Windelkerne in einem Abschnitt 14 der Maschine aufgebracht. Die aufgebrachten Windelkerne bestehen aus absorbierenden Pads und werden auf der durchlaufenden Bahn 12 positioniert. Bei den schematisch dargestellten Detektionseinrichtungen 18 und 20 handelt es sich um herkömmlich arbeitende Detektionseinrichtungen, die beispielsweise die Bahn und den aufgebrachten Windelkern optisch überwachen. Ein Mikrowellenresonator 22, 24, 26 ist entlang des Produktionsprozesses positioniert, um immer wieder eine Masse und Feuchtemessung an den Windeln und der Bahn 12 durchzuführen. Je nach Position des Mikrowellenresonators entlang der Bahn 12 können die Mikrowellenmesswerte für Masse und Feuchte in unterschiedlichen Stadien der Herstellung gemessen und überwacht werden.

Eine Einweg- oder Wegwerfwindel besteht aus einer Außenhülle aus Polyethylen (PE) und einem Saugkörper, der in der Regel aus einem Zellstoffmaterial besteht, das mit einem Superabsorber (Polymer-Salze) angereichert ist. Durch den Saugkörper kann die Flüssigkeitsmenge um das Vielfache des Eigenvolumens gebunden werden, und auch bei Druckausübung kann die Flüssigkeit gehalten werden.

Bei der Herstellung von Windeln wird in der Windelmaschine 10 eine endlose Bahn 12 der PE-Außenhülle mit einer permeablen Deckschicht in periodischen Abständen mit den Saugkörpern versehen. Die Saugkörper bestehen dabei aus einer Mischung von Zellulose und pulverförmigem Superabsorber (SAP = super absorbent polymere material).

Zur Qualitätskontrolle in der Produktion ist es wünschenswert, die Gesamtmasse und/oder ein Dichteprofil der Windelkerne mit Mikrowellenresonatoren zu messen. Aus WO 2014/170796 A1 sind eine Reihe von Vorschlägen zur Positionierung der Mikrowellenresonatoren in einer Windelmaschine bekannt. Ein Ansatz zur Auswertung der Messwerte um zuverlässige und exakte Ergebnisse zu erzielen, ist nicht bekannt.

Mit einem Mikrowellenresonator wird eine Verschiebung der Resonanzfrequenz A und eine Verbreiterung der Resonanzkurve B als Folge der dielektrischen Eigenschaften der zu untersuchenden Probe gemessen. Die Messwerte werden als Differenz der Werte des gefüllten und des leeren Resonators gebildet. Die Resonanzfrequenzverschiebung A in Hz lautet:

$$A = f_0 - f_m,$$

wobei $f_0$ die Resonanzfrequenz des leeren Resonators in Hz und $f_m$ die Resonanzfrequenz des gefüllten Resonators in Hz angibt. Für die Verbreiterung der Resonanzkurve wird auf die Vergrößerung der Halbwertsbreite der Resonanz B in Hz abgestellt. Hier gilt:

$$B = w_m - w_0,$$

wobei $w_0$ die Halbwertsbreite der Resonanz des leeren Resonators in Hz und $w_m$ die Halbwertsbreite der Resonanz des gefüllten Resonators in Hz bezeichnet.

Aus dem vorstehenden Ansatz wird deutlich, dass zu jeder Messung Informationen über die Resonanzparameter des leeren Resonators gehören. Die Leerresonanzwerte verändern sich bei Temperaturänderung und bei Verschmutzung des Resonators. Um den Einfluss der Leerresonanzwerte auf die Messwerte zu unterdrücken, werden in der Praxis folgende Maßnahmen getroffen:

- Bei Labormessungen erfolgt für jede Messwertaufnahme eine Messung der aktuellen Leerresonanzwerte.

- Bei Prozessmessungen, bei denen die Leerresonanzwerte nur selten gemessen werden können, wird der Sensor gekapselt und auf eine konstante Temperatur geregelt. Zusätzlich erfolgt regelmäßig eine Reinigung des Sensors mittels Druckluft.

Im Fall von portionierten Einheiten, die in einem endlosen, nicht metallischen Trägermaterial eingebettet sind, ist als Vorgehen in DE 10 2009 004 457 A1 der Fall beschrieben, bei dem die Abstände der portionierten Einheiten in dem Trägermaterial größer als der Durchmesser des eingesetzten Mikrowellenresonators ist. Somit ist sichergestellt, dass der Sensor zwischen den portionierten Einheiten nur das Trägermaterial erfasst. Die bekannte Auswertung setzt voraus, dass sich in periodischen Abständen nur das Trägermaterial, also die Bahn ohne portionierte Einheit in dem Messbereich befindet. Dies bedeutet, der Abstand der portionierten Einheit muss größer als das verwendete Messfeld sein.

Bei der Anwendung in der Windelmaschine ist der Abstand zwischen zwei Windelkernen häufig kleiner als der Durchmesser des Sensors. Es befindet sich also zu keinem Zeitpunkt nur das Trägermaterial allein in dem Sensor. Dies bedeutet, dass der Sensor stets zu einem gewissen Anteil Messsignale des Windelpakets enthält. Gleichwohl konnte festgestellt werden, dass bei der Anwendung von Windelkernen entlang der Bahn periodische Signalminima zwischen den Kernen auftreten. Die Erfindung beruht auf der Erkenntnis, dass bei Detektion dieser Minima anstatt eines Leerabgleichs die Signale aufgrund der Differenz zwischen den Messwerten A und B und den Messwerten der jeweiligen lokalen Minima $A_{min}$ und $B_{min}$ berechnet werden können. Somit ist es möglich, ohne Leerabgleich (wie er aus dem Stand der Technik bekannt ist) sämtliche Temperatureinflüsse auf den Sensor sowie Signalschwankungen durch Verschmutzung zu kompensieren.

Der erfindungsgemäße Auswertealgorithmus für die Mikrowellenresonatoren ist im besonderen Maße für Abstände zwischen Windelkernen geeignet, die kleiner als der Messbereich der Mikrowellenresonatoren ist. Selbstverständlich kann die erfindungsgemäße Auswertung über die lokalen Minima-Werte und deren gleitenden Mittelwert auch eingesetzt werden, wenn aufgrund einer besonderen Konfiguration der Windelmaschine die Abstände zwischen den Windelkernen größer als das verwendete Messfeld ist. Das erfindungsgemäße Verfahren ist somit universell und unabhängig von dem Abstand der Windelkerne einsetzbar.

Ein weiterer Vorteil bei Verwendung der Minimumsdetektion liegt darin, dass bei den bekannten Auswerteverfahren nach dem Stand der Technik beim Start des Messvorgangs der Windelstrang in eine vordefinierte Position relativ zu dem Sensor gefahren werden muss, d. h., der Bereich zwischen zwei Windelkernen muss sich beispielsweise im Sensor befinden. In dieser Position wird der erste Leerabgleich durchgeführt, der dann als Basis für die folgenden periodischen Leerabgleiche gilt. Diese Prozedur entfällt bei Verwendung der Minimumsdetektion, da bei Verwendung dieses Verfahrens kein Sensor - Leerabgleich benötigt wird.

Auch das aus EP 1 467 191 A1 bekannte Verfahren zur Messung von Kapseln/Tabletten setzt stets voraus, dass zwischen zwei zu messenden Tabletten das Transportband ohne Kapsel oder Tablette zur Messung eines Leerabgleichs vorliegt.

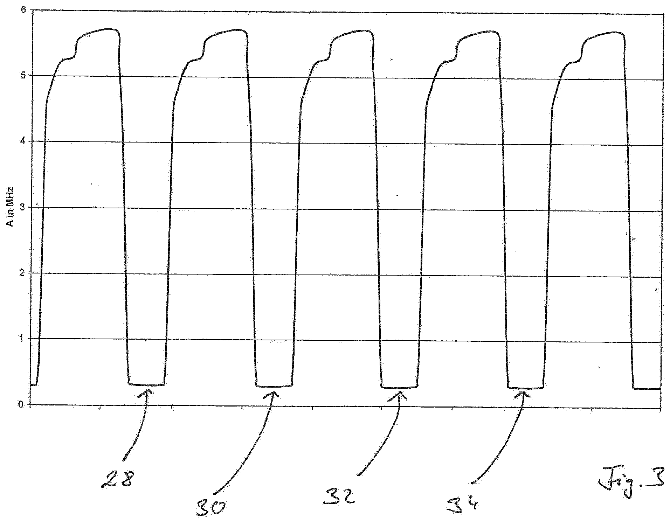

Fig. 3 zeigt den Signalverlauf der Resonanzfrequenzverschiebung A, in dem der Abstand zwischen zwei Windelkernen in Transportrichtung größer als die Ausdehnung des Messbereichs in Transportrichtung ist. Bei dieser Messsituation treten in periodischen Abständen zwischen den Windelkernen Minimumswerte 28, 30, 32, 34 auf, in denen sich nur die Bahn 12 in dem Messbereich befindet. Deutlich zu erkennen ist, dass die Messwerte für eine Konfiguration, bei der die Bahn ohne Windelkern durch den Mikrowellenresonator läuft, annähernd konstant sind und sich somit gut für einen Leerabgleich der Signalwerte ändern.

Das Verhalten der Messgrößen, insbesondere im Minimumsbereich, ändert sich deutlich in Fig. 2. Hier ist der Abstand zwischen zwei Windelkernen kleiner als der Messbereich eines Mikrowellenresonators. In Fig. 2 zu erkennen ist, dass die lokalen Minimumswerte 36 bis 50 ganz erheblich voneinander schwanken. So sind beispielsweise die lokalen Minimumswerte 40 und 42 fast um ein MHz voneinander verschieden. Deutlich wird das Ausmaß der Schwankung, wenn man hier berücksichtigt, dass die Schwankung fast bei 100 % des lokalen Minimums 40 liegt.

Die in Fig. 2 dargestellten Minimumswerte sind aufgrund der Messkonstellation so stark schwankend, dass hier eine zuverlässige Auswertung durch einen einfachen Leerabgleich bzw. Subtrahieren der Signalwerte nur zu einem sehr ungenauen Messwert führt. Eine Messung der Minima in einem festen Maschinentakt ist ebenfalls nicht möglich.

Um den Einfluss der einzelnen auftretenden Variationen der Minima-Werte 36 - 48 zu minimieren, wird erfindungsgemäß vorgeschlagen, einen gleitenden Mittelwert über die Signalminima zu bilden und diese für die Differenzbildung zu verwenden. Es wird daher für die Differenzbildung ein gleitender Mittelwert $A_{min\_AV}$ über die Signalbildung verwendet. Der gleitende Mittelwert lautet:

$$A_{min\_AV} = (A_{min\_1} + A_{min\_2} + \dots + A_{min\_N})/N$$

wobei $\{A_{min\_i}\}$ die letzten lokalen Minima für die Resonanzfrequenzverschiebung A bezeichnen. Der so gebildete gleitende Mittelwert wird zur Differenzbildung eingesetzt, so dass als Messgröße A folgender Wert verwendet wird:

$$A = A_{\_Kern} - A_{min\_AV},$$

wobei $A_{\_Kern}$ der für den Windelkern gemessene A-Wert ist. Mit den B-Werten wird analog verfahren.

Neben der vorstehend aufgeführten arithmetischen Mittelwertbildung für die A-Werte können auch andere Mittelwertbildungen verwendet werden. Insbesondere kann es von Interesse sein, auch gewichtete Mittelwerte zu verwenden, bei denen länger zurückliegende Messwerte mit einem geringeren Gewicht in die Messung eingehen, als gerade erfasste Minimumswerte.

Die mit Hilfe der Mikrowellenresonatoren bestimmten Messgrößen A, B lassen sich in unterschiedlicher Hinsicht auswerten. Da bei der Herstellung von Windeln Superabsorber als sehr hydrophiles Material eingesetzt werden, ist insbesondere auch die Bestimmung der Feuchte bzw. eines Feuchteprofils für den Windelkern von besonderem Interesse. Auch ist von Interesse, die Gesamtmasse und ein Dichteprofil für die Windeln aus den Messgrößen A, B zu bestimmen.

Die Gesamtmasse der Windelkerne erhält man durch Aufsummieren oder Integrieren der Messsignale A, B über den jeweiligen Windelkern. Teilt man den Integralwert durch die Anzahl der Einzelmessungen M, so lässt sich die Masse des Windelkerns mit folgender Kalibrationsgleichung bestimmen:

$$Masse_{Windelkern} = a_1 \cdot Int(A)/M + a_2 \cdot Int(B)/M + a_3,$$

wobei hier die Funktion "Int" für das Integral oder die Summe der Werte steht und M die Anzahl der Messung über dem Windelkern beschreibt. Die Parameter $a_1$, $a_2$ und $a_3$ können beim Kalibrieren für den weiteren Vorgang festgelegt werden. Die in der Windel enthaltene Feuchte kann durch Auswertung der Feuchtewerte mit B/A entsprechend erfolgen. Die Parameter $a_1$, $a_2$, $a_3$ sind abhängig von der Länge des Windelkerns, aber unabhängig von der Windelgeschwindigkeit, d.h der Produktions geschwindigkeit.

Bei der Bestimmung der Gesamtmasse der Windelkerne ist es grundsätzlich auch möglich, die Masse abhängig von dem Integral der Mikrowellenmesswerte zu bestimmen. Dieser Ausdruck lautet:

$$Masse' = a_1' \cdot Int(A) + a_2' \cdot Int(B) + a_3',$$

wobei $a_1'$, $a_2'$ und $a_3'$ Parameter sind, die über eine Kalibration bestimmt werden. Interessant ist hier, dass die Kalibration nur jeweils für eine Produktionsgeschwindigkeit durchgeführt werden kann, da der Wert der Integrale abhängig von der Geschwindigkeit ist. Theoretisch ist es möglich, die Geschwindigkeit, mit der die Windel durch den Sensor bewegt wird, mit in der Kalibrationsgleichung zu berücksichtigen.

In der Praxis hat sich herausgestellt, dass der Ansatz, den Mittelwert der Integralen (Int(A)/M, Int(B)/M) zu betrachten, genauere Ergebnisse liefert, da der Mittelwert der Integrale nur von der Länge der Windelkerne abhängt, die sehr wenig schwankt.

Eine weitere Besonderheit bei der Messung mit Mikrowellenresonatoren für absorbierende Hygieneartikel besteht darin, dass das bahnförmige Material unter Umständen eine große Breite besitzen kann. Bevorzugt werden für die Messung an bahnförmigem Material breite Gabelsensoren eingesetzt, wie sie beispielsweise im Textilbereich aus EP 1 316 630 B1 bekannt ist. Diese Gabelsensoren bestehen im Wesentlichen aus einem entlang seiner Längsachse halbierten Zylinder, durch den das bahnförmige Material geführt wird. Gabelsensoren besitzen eine große Feldhomogenität in Zylinderrichtung, also senkrecht zur Transportrichtung der zu messenden Bahn. Daher ist es möglich, Dichte- und Masseprofile in Bewegungsrichtung genau zu messen, wobei der Sensor einen Streifen quer zur Bewegungsrichtung integrierend misst.

Zur Erreichung der erwähnten Feldhomogenität wird in einem Gabelsensor die sogenannte Grundmode E010 betrieben. Bezeichnet man mit d den Durchmesser eines zylindrischen Gabelsensors und 1 seine Länge, so gilt für d/l → 0, dass sich die Resonanzfrequenz $f_0$ der Mode E011 zunehmend der Grundmode E010 annähert. Bei dieser Annäherung ist die Grundmode in der Praxis nicht mehr für eine Messung verwendbar, da stets Effekte und Beiträge der mitangeregten Mode E011 ihre Wirkung zeigen. Somit ist aus physikalischen Gründen die Länge von Gabelsensoren begrenzt und kann nicht beliebig skaliert oder verlängert werden. So lassen sich für Frequenzen von 2 - 3 GHz beispielsweise Gabelsensoren bis höchstens zu einer Länge von ca. 20 cm betreiben. Eine solche Messbreite kann für Hygieneartikel, beispielsweise bei Windeln für Erwachsene, zu wenig sein.

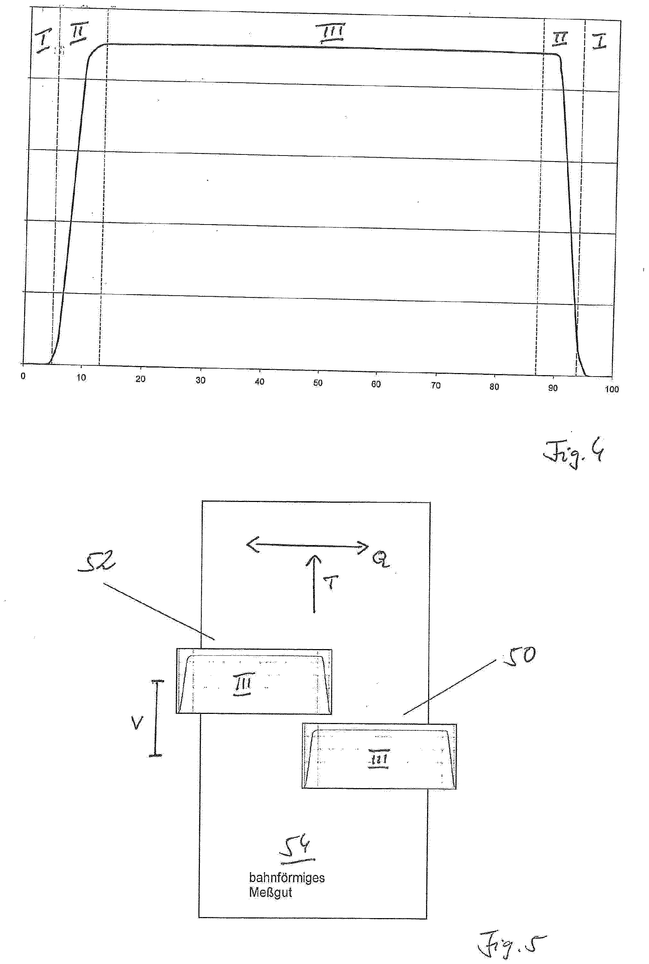

Fig. 4 zeigt die Feldstärkeverteilung in einem Gabelsensor in Richtung der Zylinderachse. Es können drei Zonen unterschieden werden:

I. Ein Feldstärkebereich außerhalb des Resonanzraums, in dem die Feldstärke exponentiell abnimmt.

II. Ein inhomogener Feldstärkebereich an Boden und Deckel der Zylinderstruktur, und

III. ein homogener Feldstärkebereich, der zur Messung von Masse und Dichte besonders gut geeignet ist.

Eine in Fig. 5 dargestellte, versetzte Anordnung der Gabelresonatoren 50, 52 erlaubt es, mit dem homogenen Feldbereichen III der beiden Gabelresonatoren 50, 52 das gesamte bahnförmige Messgut 54 zu erfassen. Die homogenen Messbereiche III der Gabelresonatoren 50, 52 sind in Querrichtung Q und in Transportrichtung T zueinander versetzt.

Der zwischen den Gabelresonatoren 50 und 52 auftretende Versatz V in Transportrichtung kann bei der Auswertung der Messsignale durch einen zeitlichen Versatz berücksichtigt werden.

Bei der Verwendung der Sensoren 50, 52 wird die Masse pro Längeneinheit durch die folgenden vier Messwerte bestimmt:

$$A_1, B_1, A_2, B_2,$$

wobei A die Resonanzfrequenzverschiebung und B die Verbreiterung der Resonanz bezeichnet, die Indizes beziehen sich auf die Resonatoren 50 und 52.

Die Masse pro Längeneinheit m in beispielsweise g/cm ist durch folgende Beziehung bestimmbar:

$$m = a_1 \cdot A_1 + a_2 \cdot A_2 + a_3 \cdot B_1 + a_4 \cdot B_2 + a_5,$$

wobei $a_i$ die Kalibrationskoeffizienten bezeichnet.

Gleichzeitig kann die Feuchte des Messguts u in % gemessen werden. Zur dichteunabhängigen Feuchtemessung wird der Quotient der Mikrowellenmesswerte verwendet. Man definiert, wie allgemein üblich, $\Phi = B/A$ oder $\Phi = \arctan(B/A)$. Die Feuchte des Messguts u in % wird unter Verwendung der Mikrowellenfeuchtewerte der beiden Resonatoren bestimmt. Es sei $\Phi_1 = \arctan(B_1/A_1)$ und $\Phi_2 = \arctan(B_2/A_2)$ für die Mikrowellenresonatoren 50, 52. Der Feuchtewert u bestimmt sich dann:

$$u = b_1 \cdot \Phi_1 + b_2 \cdot \Phi_2 + b_3,$$

wobei $b_i$ die Kalibrationskoeffizienten bezeichnet.

Aufgrund von Fertigungstoleranzen für die Gabelsensoren ergeben die Messungen in beiden Gabelresonatoren für das gleiche Produkt leicht unterschiedliche Messwerte für die Messgrößen A und B. Durch Testmessungen an einem homogenen Material können die beiden Messgrößen der Resonatoren 50, 52 in Beziehung zueinander gesetzt werden. Bei einer Verwendung eines homogenen Materials kann natürlich ein Versatz zwischen den Resonatoren entfallen. Ein Ansatz für die Auswertung der Messsignale kann daher sein:

$$A_1 = c_1 \cdot A_2,$$

$$B_1 = c_2 \cdot B_2,$$

wobei $c_1$ und $c_2$ vorbestimmte Koeffizienten sind. Der Vorteil, die Messgrößen der beiden Mikrowellenresonatoren vorab in ein konstantes Verhältnis $c_1$, $c_2$ zueinander zu setzen, besteht darin, dass für die Masse- und Feuchtewerte weniger Kalibrationskoeffizienten $a_i$ bestimmt werden müssen.

Grundsätzlich kann die vorbeschriebene Anordnung der Mikrowellenresonatoren 50, 52 auch auf mehr als zwei Mikrowellenresonatoren erweitert werden, um die zu messende Bahn mit einer möglichst homogenen Feldverteilung zu vermessen.

## Patentansprüche

1. Verfahren zur Messung von absorbierenden Hygieneartikeln, bei dem auf eine fortlaufende Bahn voneinander beabstandete Saugkörper aufgebracht werden, wobei Feuchte und/oder Dichte der Saugkörper mit mindestens einem Mikrowellenresonator mit Hilfe einer Resonanzfrequenzverschiebung (A) und einer Resonanzfrequenzverbreiterung (B) erfasst werden, wobei die Bahn durch den mindestens einen Mikrowellenresonator transportiert und fortlaufend zwei Mikrowellenmessgrößen (A, B) erfasst werden, zu deren Auswertung jeweils ein Leerwert ($A_0$, $B_0$) von dem aktuellen Messwert subtrahiert wird,
**dadurch gekennzeichnet, dass**
zur Bestimmung der Leerwerte ($A_0$, $B_0$) für jeden der Messwerte fortlaufend der Wert in einem lokalen Minimum bestimmt und mit einer Anzahl von zurückliegenden Werten für lokale Minima gemittelt wird.

2. Verfahren nach Anspruch 1, **dadurch gekennzeichnet, dass** für einen der Messwerte auch die Position des lokalen Minimums bestimmt und für den anderen Messwert der Wert für das lokale Minimum in der bestimmten Position ermittelt wird.

3. Verfahren nach Anspruch 1, **dadurch gekennzeichnet, dass** für jeden der beiden Messwerte der Wert in einem lokalen Minimum ermittelt wird.

4. Verfahren nach einem der Ansprüche 1 bis 3, **dadurch gekennzeichnet, dass** stets eine vorbestimmte Anzahl von zurückliegenden lokalen Minima gemittelt wird.

5. Verfahren nach einem der Ansprüche 1 bis 4, **dadurch gekennzeichnet, dass** mindestens zwei Mikrowellenresonatoren vorgesehen sind, die bezogen auf eine Transportrichtung der Bahn zueinander in Quer- und in Längsrichtung versetzt angeordnet sind, um die Bahn in ihrer gesamten Breite zu vermessen.

6. Verfahren nach einem der Ansprüche 1 bis 5, **dadurch gekennzeichnet, dass** jeder der Mikrowellenresonatoren seine Messwerte in Querrichtung mittelt und die gemittelten Messwerte um einen Versatz der Mikrowellenresonatoren in Transportrichtung korrigiert.

7. Verfahren nach einem der Ansprüche 1 bis 6, **dadurch gekennzeichnet, dass** jeder der Mikrowellenresonatoren einen Messbereich mit einem homogenen Feldverlauf besitzt und die mehreren Mikrowellenresonatoren derart quer zur Transportrichtung positioniert sind, dass das gesamte Band von dem Messbereich mit homogenem Feldverlauf überdeckt ist.

8. Verfahren nach einem der Ansprüche 1 bis 7, **dadurch gekennzeichnet, dass** die Masse eines Saugkörpers über die aufsummierten Messwerte der beiden Messgrößen bestimmt wird.

9. Verfahren nach einem der Ansprüche 1 bis 8, **dadurch gekennzeichnet, dass** die Feuchte eines Saugkörpers über aufsummierte Messwerte der beiden Messgrößen bestimmt wird.

10. Verfahren nach Anspruch 8 oder 9, **dadurch gekennzeichnet, dass** die aufsummierten Messwerte durch die Anzahl der Summanden geteilt wird.

**Claims**

1. Method for measuring absorbent hygiene articles, in which absorbent bodies spaced apart from one another are applied to a continuous strip, the moisture and/or density of the absorbent bodies being detected with at least one microwave resonator with the aid of a resonant frequency shift (A) and a resonant frequency broadening (B), the strip being transported through the at least one microwave resonator and two microwave measured variables (A, B) being continuously detected, for the evaluation of which a blank value ($A_0$, $B_0$) is in each case subtracted from the current measured value,
**characterized in that**
to determine the blank values ($A_0$, $B_0$) for each of the measured values, the value in a local minimum is continuously determined and averaged with a number of past values for local minima.

2. Method according to claim 1, **characterized in that** for one of the measured values the position of the local minimum is also determined and for the other measured value the value for the local minimum in the determined position is determined.

3. Method according to claim 1, **characterized in that** for each of the two measured values the value in a local minimum is determined.

4. Method according to one of claims 1 to 3, **characterized in that** a predetermined number of past local minima is always averaged.

5. Method according to one of claims 1 to 4, **characterized in that** at least two microwave resonators are provided which are arranged offset to one another in the transverse and longitudinal directions relative to a direction of transport of the strip in order to measure the strip over its entire width.

6. Method according to one of claims 1 to 5, **characterized in that** each of the microwave resonators averages its measured values in the transverse direction and corrects the averaged measured values by an offset of the microwave resonators in the transport direction.

7. Method according to one of claims 1 to 6, **characterized in that** each of the microwave resonators has a measuring region with a homogeneous field profile and the several microwave resonators are positioned transversely to the transport direction in such a way that the entire strip is covered by the measuring region with homogeneous field profile.

8. Method according to one of claims 1 to 7, **characterized in that** the mass of an absorbent body is determined via the summed measured values of the two measured variables.

9. Method according to one of claims 1 to 8, **characterized in that** the moisture of an absorbent body is determined via the summed measured values of the two measured variables.

10. Method according to claim 8 or 9, **characterized in that** the summed measured values are divided by the number of addends.

**Revendications**

1. Procédé de mesure d'articles d'hygiène absorbants, dans lequel des corps absorbants espacés les uns des autres sont déposés sur une bande continue, où l'humidité et/ou la densité des corps absorbants sont détectés avec au moins un résonateur de microondes à l'aide d'un décalage de fréquence de résonnance (A) et d'un élargissement de fréquence de résonnance (B), où la bande est transportée à travers l'au moins un résonateur de microondes et deux grandeurs de mesure de microondes (A, B) sont détectées en continu, pour l'évaluation desquelles un témoin ($A_0$, $B_0$) est respectivement soustrait de la valeur de mesure actuelle,
   **caractérisé en ce que**
   pour la détermination des témoins ($A_0$, $B_0$), pour chacune des valeurs de mesure, la valeur est déterminée en continu dans un minimum local et moyennée avec un nombre de valeurs antérieures pour des minima locaux.

2. Procédé selon la revendication 1, **caractérisé en ce que** pour l'une des valeurs de mesure, la position du minimum local est également déterminée et, pour l'autre valeur de mesure, la valeur pour le minimum local est calculée à la position déterminée.

3. Procédé selon la revendication 1, **caractérisé en ce que** pour chacune des deux valeurs de mesure, la valeur est calculée dans un minium local.

4. Procédé selon l'une des revendications 1 à 3, **caractérisé en ce qu'**un nombre prédéterminé de minima locaux antérieurs est constamment moyenné.

5. Procédé selon l'une des revendications 1 à 4, **caractérisé en ce qu'**il est prévu au moins deux résonateurs de microondes, lesquels sont installés de façon réciproquement décalée dans la direction transversale et longitudinale par rapport à une direction de transport de la bande, afin de mesurer la bande dans toute sa largeur.

6. Procédé selon l'une des revendications 1 à 5, **caractérisé en ce que** chacun des résonateurs de microondes moyenne ses valeurs de mesure dans la direction transversale et les valeurs de mesure moyennées sont corrigées selon un décalage des résonateurs de microondes dans la direction de transport.

7. Procédé selon l'une des revendications 1 à 6, **caractérisé en ce que** chacun des résonateurs de microondes possède une plage de mesure avec une évolution de champ homogène et la pluralité de résonateurs de microondes sont positionnés transversalement à la direction de transport, de telle façon que l'ensemble de la bande est couvert par la plage de mesure avec une évolution de champ homogène.

8. Procédé selon l'une des revendications 1 à 7, **caractérisé en ce que** la masse d'un corps absorbant est déterminée par les valeurs de mesure totalisées des deux grandeurs de mesure.

9. Procédé selon l'une des revendications 1 à 8, **caractérisé en ce que** l'humidité d'un corps absorbant est déterminée par des valeurs de mesure totalisées des deux grandeurs de mesure.

10. Procédé selon la revendication 8 ou 9, **caractérisé en ce que** les valeurs de mesure totalisées sont divisées par le nombre d'opérandes.

Fig. 1

Fig. 2

28    30    32    34    Fig. 3

Fig. 4

54
bahnförmiges
Meßgut

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1327876 B1
- WO 2014170796 A1
- DE 102009004457 A1
- EP 1467191 A1
- EP 1316630 B1